# EUROPEAN PATENT SPECIFICATION

(11) **EP 1 596 841 B1**
(45) Date of publication and mention of the grant of the patent: **08.10.2008**
(21) Application number: 04713281.6
(22) Date of filing: 20.02.2004
(51) Int. Cl.: A61K 9/52, A61K 9/36

(54) **THERAPEUTIC SYSTEM COMPRISING AMOXICILLIN AND CLAVULANIC ACID**
THERAPEUTISCHES SYSTEM MIT AMOXICILLIN UND CLAVULANSÄURE
SYSTEME THERAPEUTIQUE A BASE D'AMOXICILLINE ET D'ACIDE CLAVULANIQUE

(30) Priority: 21.02.2003 SI 200300044; 24.09.2003 SI 200300245
(43) Date of publication of application: 23.11.2005
(73) Proprietor: LEK Pharmaceuticals d.d., 1526 Ljubljana (SI)
(72) Inventor: KERC, Janez, 100 Ljubljana (SI); OPARA, Jerneja, 1000 Ljubljana (SI); JENKO OSEL, Maja, 4000 Kranja (SI)
(74) Representative: Kröger, Bernd
(86) International application number: PCT/SI2004/000010
(87) International publication number: WO 2004/073695

(56) References cited:
- EP-A- 0 714 656
- WO-A-02/30392
- US-A- 6 136 345
- HILTON A K ET AL: "In vitro and in vivo evaluation of an oral sustained-release floating dosage form of amoxycillin trihydrate" INTERNATIONAL JOURNAL OF PHARMACEUTICS (AMSTERDAM), vol. 86, no. 1, 1992, pages 79-88, XP001196907 ISSN: 0378-5173
- ROUGE N ET AL: "Prevention of the sticking tendency of floating minitablets filled into hard gelatin capsules" EUROPEAN JOURNAL OF PHARMACEUTICS AND BIOPHARMACEUTICS, ELSEVIER SCIENCE PUBLISHERS B.V., AMSTERDAM, NL, vol. 43, no. 2, April 1997 (1997-04), pages 165-171, XP004256873 ISSN: 0939-6411
- SINGH B N ET AL: "Floating drug delivery systems: an approach to oral controlled drug delivery via gastric retention" JOURNAL OF CONTROLLED RELEASE, ELSEVIER SCIENCE PUBLISHERS B.V. AMSTERDAM, NL, vol. 63, no. 3, 3 February 2000 (2000-02-03), pages 235-259, XP004244475 ISSN: 0168-3659
- BAUER K H ET AL: "Lehrbuch der Pharmazeutischen Technologie , PASSAGE" 1999, LEHRBUCH DER PHARMACEUTISCHEN TECHNOLOGIE, XX, XX, PAGE(S) 340 , XP002278625 figure 14.41

## Description

The present invention belongs to the field of pharmaceutical technology and relates to a novel therapeutic system suitable for once or twice-daily administration of amoxicillin and clavulanic acid. The therapeutic system comprises at least one immediate release amoxicillin/clavulanic acid composition and at least one gastroretentive pharmaceutical composition with delayed and sustained release of amoxicillin.

There is a constant need for new patient-friendly medicaments and pharmaceutical compositions which comprise amoxicillin and clavulanic acid and provide a simple and reliable method of administration and more effective treatment of bacterial infections.

Various pharmaceutical compositions comprising amoxicillin (present as trihydrate) and clavulanic acid (present as potassium clavulanate) are provided. In available compositions amoxicillin and clavulanic acid are in the ratio 2:1, 4:1, 7:1, 8:1, 14:1 and 16:1. They are intended for three- or two-times daily administration. In most examples the release of active ingredients from the pharmaceutical composition starts immediately after the composition reaches the stomach.
It is known that amoxicillin substance has the absorption window in the upper gastrointestinal tract, that is, in the stomach and in the upper small intestine.

EP 1044680 describes modified release pharmaceutical formulations comprising 1900 to 2600 mg of amoxicillin and such an amount of clavulanic acid to provide a ratio of amoxicillin to clavulanate in the range 12:1 to 20:1. They are formulated from the phase providing immediate release of clavulanic acid and a part of amoxicillin and the phase providing slow release of amoxicillin. Preferably they are multilayer tablets. The formulations described are suitable for twice daily administration.

WO 02/30392 describes formulations which allow for once daily dosing regimen of amoxicillin and clavulanic acid and provide amoxicillin concentrations greater than MIC=2 µg/ml for at least 8 hours of the dosing interval. They are suitable only for the treatment of mild infections. The single daily dose is 1700 to 2500 mg of amoxicillin and 100 to 150 mg of potassium clavulanate. The daily dose may be provided by one tablet, e.g. dispersible or chewing tablet, or several conventional tablets or capsules may be combined, of which some comprising amoxicillin and clavulanate, others only amoxicillin. The tablet containing two types of granulates is described. The immediate release granulate comprising amoxicillin trihydrate and the slow release granulate comprising crystalline sodium amoxicillin, citric acid xanthan gum and other excipients. In the stomach, this tablet disintegrates to its basic granules wherein immediate release granules are dissolved immediately and amoxicillin is absorbed. Slow-release granules are dissolved slowly, probably a part of the granules leaving the stomach before being dissolved. If undissolved granules pass the upper small intestine, where the amoxicillin absorption window is, that part of amoxicillin is not absorbed at all. Such formulation is not suitable for the treatment of infections caused by more resistant strains.

Modified release pharmaceutical formulations comprising amoxicillin and clavulanic acid are also known from the patent applications WO 95/20946, WO 95/28148, WO 96/04908, WO 94/27557 and WO 98/22091.

### Description of the invention

The present invention relates to a therapeutic system suitable for once or twice-daily administration of amoxicillin and clavulanic acid according to claim 1. One part of the dosage is provided by at least one immediate release amoxicillin/clavulanic acid pharmaceutical composition and the other part of the dosage is provided by at least one gastroretentive pharmaceutical composition with delayed and sustained release of amoxicillin.
The essence of the invention is the use of the pharmaceutical composition which is retained in the stomach and provides delayed and sustained release of amoxicillin in the stomach. Dissolved amoxicillin is absorbed from the stomach or enters the upper segment of the small intestine where it is immediately and rapidly absorbed. This way the optimal absorption of amoxicillin is achieved because practically all amoxicillin is absorbed before passing the amoxicillin absorption window. Thus, in comparison with the pharmaceutical formulations which do not provide retention in the stomach, higher bioavailability and suitable amoxicillin plasma concentrations may be achieved with even lower dosages for once or twice-daily administration. In comparison, with existing formulations, the suitable therapeutic amoxicillin plasma concentrations are achieved with the lower amoxicillin dose within a daily dosing interval.
This way, amoxicillin plasma concentrations are maintained which on average do not differ much from the Cₘₐₓ (maximum amoxicillin concentration in the blood) that would occur after administration of the immediate release dosage only which is the part of described therapeutic system. Thus, the amoxicillin concentrations in the gastrointestinal tract are in the range where no absorption saturability occurs. This way, no active substance is lost due to the absorption window and consequently with the less unabsorbed active substance fewer gastrointestinal adverse effects might be expected in respect to higher dosages which pass the absorption window.

The novel therapeutic system is more patient-friendly and is advantageous over the known pharmaceutical formulations intended for two- or three-times daily oral administration of amoxicillin and clavulanic acid. Said therapeutic system provides the simple and reliable posology and administration of the drug as directed by the physician and recommended by the manufacturer. This way the more effective treatment is provided.

The therapeutic system of the present invention is intended for the empiric treatment of mild to moderate bacterial infections caused by the bacterial strains susceptible to the combination of amoxicillin and clavulanic acid. Bacterial strains among others include gram-positive aerobic bacteria, such as *St. pneumoniae, St. pyogenes, St. viridans, St. bovis, Staph. aureus, Staph. epidermidis, Listeria* spp., *Enterococcus* spp., gram-negative aerobic bacteria, such as *H. influenzae, M. cattharalis, E. coli, Proteus* spp., *Klebsiella* spp., *N. gonorrhoeae, N. meningitidis, Pasteurella multocida* and anaerobic bacteria, such as *Peptococcus* spp., *Peptostreptococcus* spp., *Clostridium* spp., *Actinomyces israellii.*

If the therapeutic system of the invention is administered twice daily it may be used also for the empiric treatment of infections caused by bacterial strains with reduced susceptibility to amoxicillin alone, such as *St. pneumoniae,* and for the treatment of infections caused by bacterial strains susceptible to the combination of amoxicillin and clavulanic acid.

The bacteria mentioned above cause upper and lower respiratory tract infections, urinary tract infections, genital tract infections, gonorrhoea, skin and soft tissue infections, bone and connective tissue infections, cholecystitis, periodontal tissue infections, infections associated with animal or human bites, and mixed infections caused by gram-negative and gram-positive microorganisms and aerobes: chronic sinusitis and otitis media, peritonsillar abscess, breast abscess, aspiration pneumonia, peritonitis, cholangitis, postoperative intraabdominal complications, abdominal infections.

Pharmacokinetic properties of the therapeutic system of the present invention provide concentrations of amoxicillin and clavulanic acid in the plasma and consequently in the tissues which are therapeutically effective and reach or exceed minimum inhibitory concentration (MIC) of a presumed causative organism for the amoxicillin/clavulanic acid combination. The minimum inhibitory concentration (MIC) is the concentration of amoxicillin and clavulanic acid still inhibiting multiplication of the respective bacterial strain. For most isolated clinical strains the minimal inhibitory concentration for the amoxicillin/clavulanic acid combination is 1.0/0.5 µg/mL or less using the standard method for the MIC determination in the ratio amoxicillin to clavulanic acid 2:1. For bacterial strains with reduced susceptibility to amoxicillin/clavulanic acid combination the minimal inhibitory concentration is 4.0/2.0 µg/mL or less using the standard method for the MIC determination in the ratio amoxicillin to clavulanic acid 2:1.

The therapeutic system of the present invention administered once daily may provide the amoxicillin concentrations of 4.0 µg/mL at least for 7 hours within the 24 hours dosing interval. It is similar to the existing pharmaceutical amoxicillin/clavulanic acid formulations for twice or three-times daily oral administration.
The therapeutic system of the present invention administered once daily can be used for the treatment of the infections due to confirmed or suspected β-lactamase-producing pathogens susceptible to amoxicillin/clavulanic acid combination.

The therapeutic system of the present invention administered twice daily may provide the amoxicillin concentrations of 8.0 µg/mL at least for 3.5 hours within the 12 hours dosing interval. The plasma concentrations of amoxicillin achieved are above the MIC for a longer period of time than those achieved with conventional pharmaceutical formulations for twice daily administration.
The therapeutic system administered twice daily may be applicable in the case of infections due to confirmed or suspected β-lactamase-producing pathogens with reduced susceptibility to amoxicillin alone, for example in the case of *St pneumoniae* where the resistance mechanism is not β-lactamase-mediated. The suitable single dose for twice daily administration may be, for instance, about 1600 mg of amoxicillin and about 125 mg of clavulanic acid. Said dosage regimen is suitable in preventing the development of the resistance of bacteria (at least 30% of the dosing interval is above MIC=8 µg/mL, at least 40% of the dosing interval is above MIC=4 µg/mL and at least 60% of the dosing interval is above MIC=1 µg/mL or at least 60% of the dosing interval is above MIC=4 µg/mL, at least 80% of the dosing interval is above MIC=2 µg/mL and at least 90% of the dosing interval is above MIC=1 µg/mL). Said formulation is closer to the infusion which is clinically from theoretical point of view a perfect posology approach for administration of β-lactam antibiotics in comparison to administration in intervals. Said mode of administration of the novel formulation twice daily is suitable for initiation of the treatment, also known as a loading dose in the medical terminology which enables to achieve rapidly the effective concentration of the antimicrobial medicament in the blood.

A single dose of amoxicillin/clavulanic, which is administered once or twice daily is provided by a combination of one or more pharmaceutical compositions wherefrom amoxicillin and clavulanic acid are released quickly after the ingestion, and one or more pharmaceutical compositions which are retained in the stomach wherefrom amoxicillin is released slowly to provide the concentration of amoxicillin of 4.0 µg/mL for at least 7 hours and 1.0 µg/mL for at least 12 hours for once daily administration and provide the concentration of amoxicillin of 8.0 µg/mL for at least 7 hours and 1.0 µg/mL for at least 15 hours for twice daily administration.
The daily dose of amoxicillin, provided by once or twice daily administration, may preferably be from about 800 to about 6000 mg, and the dose of clavulanic acid from about 50 to about 375 mg. Amoxicillin and clavulanic acid may be in the ratio from 3:1 to 35:1. Preferred are the compositions comprising amoxicillin and clavulanic acid in the ratio 10:1 to 30:1, and most preferred in the ratio 12:1, 13:1, 22:1.

Amoxicillin may be in the form of amoxicillin trihydrate or crystalline sodium amoxicillin or as the combination thereof. Clavulanic acid may be in the form of a salt, such as potassium clavulanate.
Preferred single doses may be 1500/125, 1600/125, 2000/125, 2750/125 and 2650/80.

One or more pharmaceutical compositions providing the part of the dose for immediate release may contain from about 300 to about 2000 mg of amoxicillin and from about 50 to about 250 mg of clavulanic acid.
The part of the dose for immediate release may be provided by one or more immediate release pharmaceutical compositions selected from the group comprising a tablet, film coated tablet, rapidly disintegrating tablet, dispersible tablet, effervescent tablet, chewing tablet, capsule, single dose sachet comprising granulate, suspension and pellets. It is possible to combine more compositions which may be the same or different, of the same or different dosage. For example, one or more tablets 500/125, 875/125, 500/62.5, 1000/80, 1000/125, 1050/125 mg, a dispersible tablet 500/125, 875/125 1000/125, 1000/80, 1050/125 mg may be used, or the combinations thereof. Some tablets may comprise amoxicillin and clavulanic acid, others amoxicillin only. The use of one immediate release amoxicillin/clavulanic acid pharmaceutical combination is preferred.

The immediate release amoxicillin/clavulanic acid pharmaceutical composition may also comprise absorption enhancers which improve amoxicillin absorption from the gastrointestinal tract. Suitable absorption enhancers may be surface active agents, fatty acids, middle-chain glycerides, steroid detergents (bile acid salts), acyl carnitine and alkanoyl cholines (carnitine and choline esters with middle- and long-chain fatty acid), N-acyl derivatives of α-amino acids, N-acyl derivatives of non-α-amino acids, chitosans and other mucoadhesive polymers. Especially suitable absorption enhances may be, for instance, sodium deoxycholate, sodium taurocholate, polysorbate 80, sodium lauryl sulfate, sodium dodecylsulfate, octanoic acid, sodium docusate, sodium laurate, glyceryl monolaurate, stearic acid, palmitic acid, palmitoleic acid, glyceryl monooleate, ethylenediaminotetraacetic acid, sodium edetate, sodium citrate, β-cyclodextrine and sodium salicylate. Preferred absorption enhancers are sodium deoxycholate, sodium docusate and sodium lauryl sulfate.

The part of the dose for delayed and sustained release of amoxicillin is provided by one or more pharmaceutical gastroretentive compositions which are retained in the stomach. Suitable gastroretentive pharmaceutical compositions may be, for instance, floating pharmaceutical compositions, heavy pharmaceutical compositions, bioadhesive pharmaceutical compositions, expandable pharmaceutical compositions, pharmaceutical compositions which retain in the stomach due to their specific form and cannot pass through the pylorus into the duodenum and the like. Suitable floating pharmaceutical compositions may be, for instance, a floating capsule or a floating tablet, preferably, a floating capsule. The part of the dose for delayed and sustained release may be divided between more than one floating capsules or tablets. The said part preferably comprises from 500 to 2000 mg of amoxicillin. Optionally, the gastroretentive pharmaceutical composition may also comprise clavulanic acid.
The dose of 1500 mg of amoxicillin and 125 mg of clavulanic acid may be provided, for instance, by one film coated tablet 500/125 and two floating capsules each comprising 500 mg of amoxicillin. The dose of 1600 mg of amoxicillin and 125 mg of clavulanic acid, may be provided, for instance, by one film coated tablet 500/125 and two floating capsules each comprising 550 mg of amoxicillin, the dose of 2650 mg of amoxicillin and 80 mg of clavulanic acid, for instance, by one dispersible tablet 1000/80 and three floating capsules each comprising 550 mg of amoxicillin. The dose of 2525 mg of amoxicillin and 125 mg of clavulanic acid may be provided, for instance, by one film coated tablet 875/125 and three floating capsules each comprising 550 mg of amoxicillin. The dose of 2000 mg of amoxicillin and 125 mg of clavulanic acid may be provided, for instance, by one film coated tablet 500/125 and three floating capsules each comprising 500 mg of amoxicillin.
Further the dose of 1550 mg of amoxicillin and 125 mg of clavulanic acid, may be provided, for instance, by one film coated tablet 1000/125 and one floating capsule comprising 550 mg of amoxicillin. The dose of 1600 mg of amoxicillin and 125 mg of clavulanic acid, may be provided, for instance, by one film coated tablet 1050/125 and one floating capsule comprising 550 mg of amoxicillin. The dose of 2000 mg of amoxicillin and 125 mg of clavulanic acid may be provided, for instance, by one film coated tablet 900/125 and two floating capsules each comprising 550 mg of amoxicillin.

The present invention also relates to a delayed and sustained release amoxicillin floating capsule (gastroretentive composition) used in the therapeutic system of the present invention to provide for the daily dose in the combination with an immediate release amoxicillin/clavulanic acid pharmaceutical composition. The release of amoxicillin is delayed for about one hour being defined by dissolving of the capsule cap or the coating of the capsule.

The floating, capsule of the invention comprises a coated capsule body, coated or uncoated capsule cap, the granulate and at least one tablet wherein the granulate and the tablet comprise amoxicillin. Amoxicillin may be in the form of trihydrate or crystalline sodium amoxicillin or the combination thereof. The capsule body and the cap basic material may be a polymer material such as, e.g., hydroxypropylmethylcellulose (HPMC), gelatine and starch. Preferably, the capsule is made from hydroxypropylmethylcellulose.
The capsules may be wholly coated, or only a capsule body may be coated. A body and a cap may be coated with the same or different coating. If coatings are different, a cap coating must be dissolved prior to a capsule body coating.
Suitable coatings may be well soluble, poorly soluble, or slowly dissolving coatings. Suitable soluble or insoluble polymers, or combinations of insoluble polymers with soluble polymers may be, e.g., combinations of ethylcellulose and hydropropylmethylcellulose, hydroxypropylcellulose, hydroxyethylcellulose, methylcellulose or polyvinylpyrrolidone, combinations of methacrylate/trimethylammonioethylmethacrylate copolymers (e.g., Eudragit RL PO, Eudragit RL 100, Eudragit RL30D, Eudragit RS PO, Eudragit RS 100, Eudragit RS30D or combinations thereof) and hydroxypropylmethylcellulose, hydroxypropylcellulose, hydroxyethylcellulose or methylcellulose, combinations of a neutral polymer of methacrylate (e.g., Eudragit NE 30 D, Eudragit NE 40 D) and hydroxypropylmethylcellulose, hydroxypropylcellulose, hydroxyethylcellulose, methylcellulose or polyvinylpyrrolidone.
The coatings may further comprise other excipients conventionally used in the coatings such as fillers, e.g., talc, lactose, polysaccharides and the like, plasticizers, e.g., dibutyl sebacate, triethyl citrate, polyethylene glycol, adipic acid, coconut oil, oleic acid and the like, colorants, e.g., titanium dioxide, lakes, pigments and the like, antioxidants and other excipients. The coating may also comprise bioadhesive polymers.

A particularly suitable coating is the combination of ethylcellulose and hydropropylcellulose or the combination of Surelease® and hydroxypropylmethylcellulose in the ratio from 70:30, 60:40 to 50:50. Surelease® is Colorcon's trade mark for the aqueous dispersion of ethylcellulose.

Prior to application of the functional coating, the capsule or capsule parts may be coated with a dispersion (solution or suspension) of a hydrophilic polymer, e.g., hydroxypropylmethylcellulose, hydroxypropylcellulose, hydroxyethylcellulose.

The capsules or their parts may be coated empty (before filling) or the capsules already filled with the granulate and tablets are coated. The amount of the coating to be used may be from 0.5 to 10 mg/cm² if coated prior to filling or from 0.5 to 30 mg/cm² if the capsules already filled with granulate and tablets are coated. The suitable coat thickness may be from 2 to 200 µm, preferably 10 to 50 µm if coated before filling and 2 to 600 µm if the capsules already filled with granulate and tablets are coated, respectively.

Sustained release may be achieved with the type of the coating or a combination of the coated body and uncoated cap or a combination of different coatings.

The coatings may be applied by the techniques which are conventional for capsule or tablet coating in pharmaceutical technology. The coating dispersion may be a solution or suspension of polymers and other excipients. Suitable solvents for the preparation of the coating dispersion may be, for instance, water, ethanol, methanol, propan-2-ole, acetone, ethyl acetate, acetic acid, glycols, dichloromethane, dimethylformamide, dimethysulfoxide, chloroform, toluene, methylene chloride, benzene, ethoxyethyl acetate, ethylene glycol monoacetate, ethyl lactate, monoethyl acetate, methylethyl ketone and combinations thereof.
The capsules or their parts, that is, the capsule body or cap, in addition to the first functional coating, may be also coated with the second functional coating which by the composition may be the same or different from the first coating. For the second coating, soluble or insoluble polymers or a combination of insoluble and soluble polymers may be used. Other excipients may be the same or different from the excipients of the first coating. The procedures for preparing the second functional coating may be the same as the procedures for preparing the first functional coating.

The granulate comprises amoxicillin and at least one release-control hydrophilic or lipophilic substance. Polymer or nonpolymer substances may be used. Suitable polymer substances may be selected from a group comprising hydroxypropylcellulose, hydroxypropylmethylcellulose, methylcellulose, ethylcellulose, hydroxyethylcellulose, sodium carboxymethylcellulose, cellulose acetate phthalate, polyvinyl acetate phthalate, hydroxymethylcellulose phthalate, polyvinyl alcohol, polyvinylpyrrolidone, methylhydroxyethylcellulose, polymers and copolymers of acrylic and methylacrylic acid, copolymers of ethylacrylate and methylacrylate, maltodextrin, xanthan gum, guar gum, acacia, alginic acid and sodium alginate. Suitable nonpolymers may be carnauba wax, cetyl alcohol, hydrogenated vegetable oil, hydrogenated castor oil, glycerol monostearate, glycerol palmitostearate, a mixture of mono-, di- and triglycerides and the like.
Preferably hydroxypropylmethylcellulose, methylcellulose and ethylcellulose are used.

The granulate may further comprise other excipients, for instance, different fillers, binders, disintegrants, glidants, lubricants and absorption enhancers of amoxicillin from the gastrointestinal tract. Suitable fillers may be microcrystalline cellulose, powdered cellulose, lactose, starch, pregelatinized starch, saccharose, glucose, mannitol, sorbitol, calcium phosphate, calcium hydrogen phosphate, aluminium silicate, sodium chloride, potassium chloride, calcium carbonate, calcium sulfate, dextrates, dextrin, maltodextrin, glycerol palmitostearate, hydrogenated vegetable oil, kaolin, magnesium carbonate, magnesium oxide, polymethacrylates, talc and the like, preferably microcrystalline cellulose and lactose. Suitable binders may be starch, pregelatinized starch, gelatine, sodium carboxymethylcellulose, polyvinylpyrrolidone, alginic acid, sodium alginate, acacia, carbomer, dextrin, ethylcellulose, guar gum, hydrogenated vegetable oil, methylcellulose, hydroxyethylcellulose, hydroxypropylcellulose, hydroxypropylmethylcellulose, glucose syrup, magnesium aluminium silicate, maltodextrin, polymethacrylates, zein, preferably hydroxypropylcellulose, hydroxypropylmethylcellulose and polyvinylpyrrolidone. Suitable disintegrants may be selected from starch, pregelatinized starch, sodium starch glycolate, sodium carboxymethylcellulose, cross-linked sodium carboxymethylcellulose, calcium carboxymethylcellulose, methylcellulose, microcrystalline cellulose, powdered cellulose, polacrilin potassium, cross-linked polyvinylpyrrolidone, alginic acid, sodium alginate, colloidal silicon dioxide, guar gum, magnesium aluminium silicate and the like, preferably sodium starch glycolate, cross-linked sodium carboxymethylcellulose and cross-linked polyvinylpyrrolidone. Suitable glidands may be magnesium stearate, calcium stearate, aluminium stearate, stearic acid, palmitic acid, cetanol, stearol, polyethylene glycols of different molecular weights, magnesium trisilicate, calcium phosphate, colloidal silicon dioxide, talc, powdered cellulose, starch and the like, preferably colloidal silicon dioxide. Suitable lubricants may be stearic acid, calcium magnesium, zinc or aluminium stearate, siliconized starch, glycerol monostearate, glycerol palmitostearate, hydrogenated castor oil, hydrogenated vegetable oil, mineral oil, light mineral oil, polyethylene glycol, sodium benzoate, sodium lauryl sulfate, sodium stearyl fumarate, talc and the like. Preferred lubricants are calcium or magnesium stearate and stearic acid.
Suitable absorption enhancers of amoxicillin may be selected from suitable surface active agents, fatty acids, middle-chain glycerides, steroid detergents (bile acid salts), acyl carnitine and alkanoyl cholines (carnitine and choline esters with middle- and long-chain fatty acid), N-acyl derivatives of α-amino acids, N-acyl derivatives of non-α-amino acids, chitosans and other mucoadhesive polymers. Especially suitable absorption enhances may be, for instance, sodium deoxycholate, sodium taurocholate, polysorbate 80, sodium lauryl sulfate, sodium dodecylsulfate, octanoic acid, sodium docusate, sodium laurate, glyceryl monolaurate, stearic acid, palmitic acid, palmitoleic acid, glyceryl monooleate, sodium taurocholate, ethylenediaminotetraacetic acid, sodium edetate, sodium citrate, β-cyclodextrine and sodium salicylate. Preferred absorption enhancers are sodium deoxycholate, sodium docusate and sodium lauryl sulfate.
The granulate may be prepared by the techniques conventionally used in the pharmaceutical art for the preparation of the granulates, for example, simple mixing of the blend of powders (direct mix) and dry or wet granulation. For dry granulation, for instance, the slugging or compacting procedure may be used. A suitable solvent for wet granulation may be, for instance, water, ethanol, methanol, propan-2-ole, acetone, ethyl acetate, acetic acid, glycols, dichloromethane, dimethylformamide, dimethylsulfoxide, chloroform, toluene, methylene chloride, benzene, ethoxyethyl acetate, ethylene glycol monoacetate, ethyl lactate, monoethyl acetate, methylethyl ketone and combinations thereof.

The tablet comprises amoxicillin and excipients which are the same as in the granulate, or may be different. The tablet composition may be qualitatively and expressed by percentage the same or different from the granulate composition. Optionally, the tablet may be prepared only from excipients without amoxicillin.

The floating capsule of the present invention may include one or more tablets. By composition they may be the same or different. The tablet comprising amoxicillin may be combined with tablets without amoxicillin, tablets with the composition being the same of different from the composition of the granulate.

The tablet comprises at least one release-control hydrophilic or lipophilic substance to control the release. Polymer or nonpolymer substances may be used. Suitable polymer substances may be hydroxypropylcellulose, hydroxypropylmethylcellulose, methylcellulose, ethylcellulose, hydroxyethylcellulose, sodium carboxymethylcellulose, cellulose acetate phthalate, polyvinyl acetate phthalate, hydroxymethylcellulose phathalate, polyvinyl alcohol, polyvinylpyrrolidone, methylhydroxyethylcellulose, polymers and copolymers of acrylic and methylacrylic acid, copolymers of ethylacrylate and methylacrylate, maltodextrin, xanthan gum, guar gum, acacia, alginic acid and sodium alginate. Nonpolymers may be carnauba wax, cetyl alcohol, hydrogenated vegetable oil, hydrogenated castor oil, glycerol monostearate, glycerol palmitostearate, a mixture of mono-, di- and triglycerides and the like.
Preferably hydroxypropylmethylcellulose, methylcellulose and ethylcellulose are used.

The tablet may also comprise other excipients, for instance, fillers, binders, disintegrants, surfactants, glidants, lubricants and absorption enhancers of amoxicillin from the gastrointestinal tract. Suitable fillers may be microcrystalline cellulose, powdered cellulose, lactose, starch , pregelatinized starch, saccharose, glucose, mannitol, sorbitol, calcium phosphate, calcium hydrogen phosphate, aluminium silicate, sodium chloride, potassium chloride, calcium carbonate, calcium sulfate, dextrates, dextrin, maltodextrin, glycerol palmitostearate, hydrogenated vegetable oil, kaolin, magnesium carbonate, magnesium oxide, polymethacrylates, talc and the like, preferably microcrystalline cellulose and lactose. Suitable binders may be starch, pregelatinized starch, gelatine, sodium carboxymethylcellulose, polyvinylpyrrolidone, alginic acid, sodium alginate, acacia, carbomer, dextrin, ethylcellulose, guar gum, hydrogenated vegetable oil, methylcellulose, hydroxyethylcellulose, hydroxypropylcellulose, hydroxypropylmethylcellulose, glucose syrup, magnesium aluminium silicate, maltodextrin, polymethacrylates, zein, preferably hydroxypropylcellulose, hydroxypropylmethylcellulose and polyvinylpyrrolidone. Suitable disintegrants may be starch, pregelatinized starch, sodium starch glycolate, sodium carboxymethylcellulose, cross-linked sodium carboxymethylcellulose, calcium carboxymethylcellulose, methylcellulose, microcrystalline cellulose, powdered cellulose, polacrilin potassium, cross-linked polyvinylpyrrolidone, alginic acid, sodium alginate, colloidal silicon dioxide, guar gum, magnesium aluminium silicate and the like, preferably sodium starch glycolate, cross-linked sodium carboxymethylcellulose and cross-linked polyvinylpyrrolidone. Suitable glidands may be magnesium stearate, calcium stearate, aluminium stearate, stearic acid, palmitic acid, cetanol, stearol, polyethylene glycols of different molecular weights, magnesium trisilicate, calcium phosphate, colloidal silicon dioxide, talc, powdered cellulose, starch and the like preferably colloidal silicon dioxide. Suitable lubricants may be stearic acid, calcium magnesium, zinc or aluminium stearate, siliconized starch, glycerol monostearate, glycerol palmitostearate, hydrogenated castor oil, mineral oil, light mineral oil, polyethylene glycol, sodium benzoate, sodium lauryl sulfate, sodium stearyl fumarate, talc and the like. Preferred lubricants are calcium or magnesium stearate and stearic acid. Suitable absorption enhancers of amoxicillin may be selected from suitable surfactants, fatty acids, middle-chain glycerides, steroid detergents (bile acid salts), acyl carnitine and alkanoyl cholines (carnitine and choline esters with middle- and long-chain fatty acid), N-acyl derivatives of α-amino acids, N-acyl derivatives of non-α-amino acids, chitosans and other mucoadhesive polymers. Especially suitable absorption enhances are, for instance, sodium deoxycholate, sodium taurocholate, polysorbate 80, sodium lauryl sulfate, sodium dodecylsulfate, octanoic acid, sodium laurate, glyceryl monolaurate, stearic acid, palmitic acid, palmitoleic acid, glyceryl monooleate, sodium taurocholate, ethylenediaminotetraacetic acid, sodium edetate, sodium citrate, β-cyclodextrine and sodium salicylate. Preferred absorption enhancers are sodium deoxycholate, sodium docusate and sodium lauryl sulfate. The tablets may be prepared by the techniques known in the pharmaceutical technology, that is, by direct tabletting the blend of powders or by tabletting the granulate prepared by wet or dry granulation. For dry granulation, for instance, the slugging or compacting procedure is used. A suitable solvent for wet granulation may be, for instance, water, ethanol, methanol, propan-2-ole, acetone, ethyl acetate, acetic acid, glycols, dichloromethane, dimethylformamide, dimethylsulfoxide, chloroform, toluene, methylene chloride, benzene, ethoxyethyl acetate, ethylene glycol monoacetate, ethyl lactate, monoethyl acetate, methylethyl ketone and combinations thereof.

Absorption enhancers of amoxicillin from the gastrointestinal tract may be in the granulate and/or in the tablet of the floating pharmaceutical composition. They may be either in the immediate release pharmaceutical composition, or in the floating pharmaceutical composition or in the both.

The present invention further relates to the method of treatment of bacterial infections comprising once or twice daily administration of a therapeutic system comprising at least one immediate release amoxicillin/clavulanic acid pharmaceutical composition and at least one delayed and sustained release amoxicillin pharmaceutical composition which is retained in the stomach. Said therapeutic system provides in 24 hours the amoxicillin plasma concentration of >4.0 µg/mL for at least 7 hours and >1.0 µg/mL for at least 12 hours for once daily administration and provide the concentration of amoxicillin of 8.0 µg/mL for at least 7 hours and 1.0 µg/mL for at least 15 hours for twice daily administration being greater than the MIC for the majority of strains which are susceptible to the amoxicillin/clavulanic acid combination. The therapeutic system of the present invention administered once daily may be used for the treatment of the infections due to confirmed or suspected β-lactamase-producing pathogens susceptible to amoxicillin/clavulanic acid combination or may be administered twice daily in the case of infections due to confirmed or suspected β-lactamase-producing pathogens with reduced susceptibility to amoxicillin alone, for example in the case of *St pneumoniae* where the resistance mechanism is not β-lactamase-mediated.
The invention further relates to a therapeutic system which provides the daily dose of amoxicillin without clavulanic acid whereat the part of the daily dose is at least one immediate release amoxicillin pharmaceutical composition and the delayed and sustained release of amoxicillin is provided by at least one pharmaceutical composition which is retained in the stomach and is preferably a floating capsule.

The therapeutic system of said invention, that is, at least one immediate release amoxicillin/clavulanic acid pharmaceutical composition and at least one delayed and sustained release amoxicillin pharmaceutical composition which is retained in the stomach and represents a single dose, is packed in a separate part of a blister. Single doses are separated by the blister perforation and clearly labelled with consecutive days from the start of therapy.

The present invention is illustrated by the following examples:

### Example 1:

The therapeutic system with daily dose of 1600 mg of amoxicillin and 125 mg of clavulanic acid (1600/125) was provided by one conventional film coated tablet comprising 500 mg of amoxicillin in the form of trihydrate and 125 mg of clavulanic acid in the form of potassium clavulanate (Amoksiklav® 500/125) and two floating capsules each comprising of 550 mg of amoxicillin in the form of trihydrate.

**Composition of the floating capsule:**

| Ingredients | Weight |
|---|---|
| Granulate: | |
| Amoxicillin trihydrate | 481.76 mg |
| Hydroxypropylmethylcellulose (HPMC, Methocel K100LV) | 36.32 mg |
| Microcrystalline cellulose(Avicel PH102) | 36.32 mg |
| Mg stearate | 5.60 mg |
| Total weight of granulate | 560.00 mg |
| | |
| Tablet: | |
| Amoxicillin trihydrate | 154.84 mg |
| Hydroxypropylmethylcellulose (Methocel K100LV) | 11.68 mg |
| Microcrystalline cellulose(Avicel PH102) | 11.68 mg |
| Mg stearate | 1.80 mg |
| Total weight of tablet | 180.00 mg |
| | |
| Capsule: HPMC size 00 | |
| Coating of capsule body: Surelease:HPMC E6 60:40 | 6.510 mg/cm² |
| Capsule cap uncoated | |

### Method of preparation:

Amoxicillin trihydrate, Methocel, Avicel and magnesium stearate were homogeneously mixed to prepare the granulate.
Amoxicillin trihydrate, Methocel, Avicel and magnesium stearate were homogeneously mixed and the resulting granulate was compressed into tablets each weighing 180 mg.
HPMC E6 was dissolved in water for 45 minutes and Surelease was suspended in the HPMC solution with constant stirring for 10 minutes to obtain a 10% suspension. The suspension was sprayed over the HPMC capsule bodies in a perforated coating pan at a temperature at about 40°C to obtain the suitable amount of the coating.

The coated capsule bodies were filled with 560 mg of the granulate and the tablet, and closed with the uncoated capsule cap.

### Example 2:

The therapeutic system with daily dose of 2000 mg of amoxicillin and 125 mg of clavulanic acid (2000/125) was provided by one film coated tablet Amoksiklav^{®} 500/125 and three floating capsules each comprising 550 mg of amoxicillin in the form of trihydrate.

**Composition of the floating capsule:**

| ingredients | Weight |
|---|---|
| Granulate: | |
| Amoxicillin trihydrate | 448.0 mg |
| Hydroxypropylmethylcellulose (Methocel K100LV) | 53.2 mg |
| Microcrystalline cellulose (Avicel PH102) | 53.2 mg |
| Mg stearate | 5.6 mg |
| Total weight of granulate | 560.0 mg |
| | |
| Tablet | |
| Amoxicillin trihydrate | 144.0 mg |
| Hydroxypropylmethylcellulose (Methocel K100LV) | 17.1 mg |
| Microcrystalline cellulose (Avicel PH102) | 17.1 mg |
| Mg stearate | 1.8 mg |
| Total weight of tablet | 180.0 mg |
| | |
| Capsule: HPMC size 00 | |
| Coating of capsule body: Surelease:HPMC E6 60:40 | 6.510 mg/cm² |
| Capsule cap uncoated | |

### Method of preparation:

Amoxicillin trihydrate, Methocel, Avicel and magnesium stearate were homogeneously mixed to prepare the granulate.
Amoxicillin trihydrate, Methocel, Avicel and magnesium stearate were homogeneously mixed and the resulting granulate was compressed into tablets each weighing 180 mg.
HPMC E6 was dissolved in water for 45 minutes and Surelease was suspended in the HPMC solution with constant stirring for 10 minutes to obtain a 10% suspension. The suspension was sprayed over the HPMC capsule bodies in a perforated coating pan at a temperature at about 40°C to obtain the suitable amount of the coating.
The coated capsule bodies were filled with 560 mg of the granulate and the tablet, and closed with the uncoated capsule cap.

### Example 3:

### A pharmacokinetic evaluation of the therapeutic system 1600/125 mg:

Twelve healthy male volunteers, aged 18-45 years, with body weight in the range ±10% of ideal body weight were included in a crossover study with a single dose administration of the drug in fed conditions. In one period volunteers received one immediate-release Amoksiklav® tablet 500mg/125mg and in the other period they received the therapeutic system for once-daily administration of 1600mg/125mg (1 tablet of Amoksiktav® 500mg/125mg and 2 floating capsules each comprising 550 mg of amoxicillin). There was a 7-day wash-out period between the two periods. Blood sampling time were the following: pre-dose, and 0.25, 0.5, 0.75, 1, 1.25, 1.5, 1.75, 2, 2.5, 3, 4, 5, 6, 7, 8, 9, 10, 11, 12, 14 and 24 h after dosing, that is, 22 samples per subject per period.
Amoxicillin and clavulanic acid concentrations were determined in the plasma. By comparing the plasma concentration profiles of amoxicillin of both medicaments, the contribution of the floating capsules in the therapeutic system for once-daily administration was defined. The time above the MIC =1 µg/mL of amoxicillin and the time above the MIC=4 µg/mL of the treatment system for once-daily administration of amoxicillin and clavulanic acid of 1600mg/125mg were determined in the study.

### Results of the study:

The time above the MIC=1 µg/mL: 12.5 h and the time above the MIC=4 µg/mL: 7.88 h, indicating 52.1% (for MIC=1 µg/mL) and 32.8% (for MIC=4 µg/mL) of the 24-hour dosing interval. With respect to clavulanic acid both medicaments are bioequivalent.

### Example 4:

The therapeutic system with single dose of 1550 mg of amoxicillin and 125 mg of clavulanic acid (1550/125) was provided by one conventional film coated tablet comprising 1000 mg of amoxicillin in the form of trihydrate and 125 mg of clavulanic acid in the form of potassium clavulanate and one floating capsule comprising 550 mg of amoxicillin in the form of trihydrate.

**Composition of the tablet 1000/125:**

| Ingredients | Weight |
|---|---|
| Amoxicillin trihydrate | 1164.14 mg |
| Potassium clavulanate | 150.42 mg |
| Microcrystalline cellulose(Avicel PH102 dried) | 119.04 mg |
| Polyplasdone XL dried | 70.00 mg |
| Sodium lauryl sulfate | 25.00 mg |
| Cross-linked sodium carboxymethylcellulose | 36.00 mg |
| Colloidal silicon dioxide | 12.00 mg |

| Mg stearate | 23.40 mg |
|---|---|
| Hydroxypropylcellulose | 22.12 mg |
| Ethylcellulose | 1.08 mg |
| Triethyl citrate | 1.22 mg |
| Talc | 2.68 mg |
| Titanium dioxide | 11.70 mg |
| Polisorbat 80 | 1.20 mg |
| Total weight of tablet | 1640.00 mg |

### Method of preparation:

Amoxicillin trihydrate, potassium clavulanate, microcrystalline cellulose, sodium lauryl sulphate, a part of polyplasdone XL, cross-linked sodium carboxymethylcellulose, colloidal silicon dioxide, and Mg stearate were mixed and slugged on rotary tabletting machines, then a part of a part of polyplasdone XL, cross-linked sodium carboxymethylcellulose, colloidal silicon dioxide, and Mg stearate were added, mixed and so prepared granulation was compressed into tablets each weighing 1600mg.
Ethanolic dispersion of hydroxypropylcellulose, ethylcellulose, triethyl citrate, talc, titanium dioxide and polisorbat was prepared using laboratory mixer and tablet cores were coated with the so prepared dispersion in a coating pan until 40mg of coating was applied.

**Composition of the floating capsule:**

| Ingredients | Weight |
|---|---|
| Granulate: | |
| Amoxicillin trihydrate | 487.95 mg |
| Hydroxypropylmethylcellulose (HPMC, Methocel K100LV) | 36.32 mg |
| Microcrystalline cellulose(Avicel PH102) | 30.13 mg |
| Sodium lauryl sulfate | 25.00 mg |
| Mg stearate | 5.60 mg |
| Total weight of granulate | 585.00 mg |
| | |
| Tablet: | |
| Amoxicillin trihydrate | 154.84 mg |
| Hydroxypropylmethylcellulose (Methocel K100LV) | 9.18 mg |
| Microcrystalline cellulose(Avicel PH102) | 9.18 mg |
| Sodium lauryl sulfate | 25.00 mg |
| Mg stearate | 1.80 mg |
| Total weight of tablet | 200.00 mg |
| | |
| Capsule: HPMC size 00 | |
| Precoated capsule: EC:HPC 40:60 | 3.267 mg/cm² |

### Method of preparation:

Amoxicillin trihydrate, Methocel, Avicel, sodium lauryl sulfate and magnesium stearate were homogeneously mixed to prepare the granulate.
Amoxicillin trihydrate, Methocel, Avicel, sodium lauryl sulfate and magnesium stearate were homogeneously mixed and the resulting granulate was compressed into tablets each weighing 200 mg.
Ethanolic dispersion of hydroxypropylcellulose, ethylcellulose, triethyl citrate, and talc was prepared using laboratory mixer and preclosed capsules were coated with the so prepared dispersion in a perforated coating pan at about 30°C to obtain the suitable amount of the coating.
The suspension was sprayed over the HPMC capsule bodies in a perforated coating pan at a temperature at about
The coated capsules were opened, filled with 560 mg of the granulate and the tablet, and closed.

### Example 5:

The therapeutic system with single dose of 1600 mg of amoxicillin and 125 mg of clavulanic acid (1600/125) was provided by one film coated tablet 1050/125mg and one floating capsule comprising 550 mg of amoxicillin in the form of trihydrate.

**Composition of a tablet 1050/125mg:**

| Ingredients | Weight |
|---|---|
| Amoxicillin trihydrate | 1220.93 mg |
| Potassium clavulanate | 150.60 mg |
| Silicified microcrystalline cellulose(Prosolv dried) | 62.07 mg |
| Polyplasdone XL dried | 70.00 mg |
| Sodium lauryl sulfate | 25.00 mg |
| Cross-linked sodium carboxymethylcellulose | 36.00 mg |
| Colloidal silicon dioxide | 12.00 mg |
| Mg stearate | 23.40 mg |
| Hydroxypropylcellulose | 22.12 mg |
| Ethylcellulose | 1.08 mg |
| Triethyl citrate | 1.22 mg |
| Talc | 2.68 mg |
| Titanium dioxide | 11.70 mg |
| Polisorbat 80 | 1.20 mg |
| Total weight of tablet | 1640.00 mg |

### Method of preparation:

Amoxicillin trihydrate, potassium clavulanate, silicified microcrystalline cellulose, sodium lauryl sulphate, a part of polyplasdone XL, cross-linked sodium carboxymethylcellulose, colloidal silicon dioxide, and Mg stearate were mixed and slugged on rotary tabletting machines, then a part of a part of polyplasdone XL, cross-linked sodium carboxymethylcellulose, colloidal silicon dioxide, and Mg stearate were added, mixed and so prepared granulation was compressed into tablets each weighing 1600mg.
Ethanolic dispersion of hydroxypropylcellulose, ethylcellulose, triethyl citrate, talc, titanium dioxide and polisorbat was prepared using laboratory mixer and tablet cores were coated with the so prepared dispersion in a coating pan until 40mg of coating was applied.

**Composition of the floating capsule:**

| | |
|---|---|
| Ingredients | Weight |
| Granulate: | |
| Amoxicillin trihydrate | 487.95 mg |
| Hydroxypro,pylmethylcellulose (HPMC, Methocel K100LV) | 36.32 mg |
| Microcrystalline cellulose(Avicel PH102) | 30.13 mg |
| Sodium lauryl sulfate | 25.00 mg |
| Mg stearate | 5.60 mg |
| Total weight of granulate | 585.00 mg |
| | |
| Tablet: | |
| Amoxicillin trihydrate | 154.84 mg |
| Hydroxypropylmethylcellulose (Methocel K100LV) | 9.18 mg |
| Microcrystalline cellulose(Avicel PH102) | 9.18 mg |
| Sodium lauryl sulfate | 25.00 mg |
| Mg stearate | 1.80 mg |
| Total weight of tablet | 200.00 mg |
| | |
| Capsule: HPMC size 00 | |
| Precoated capsule: EC:HPC 40:60 | 3.267 mg/cm² |

### Method of preparation:

Amoxicillin trihydrate, Methocel, Avicel, sodium lauryl sulfate and magnesium stearate were homogeneously mixed to prepare the granulate.
Amoxicillin trihydrate, Methocel, Avicel, sodium lauryl sulfate and magnesium stearate were homogeneously mixed and the resulting granulate was compressed into tablets each weighing 200 mg.
Ethanolic dispersion of hydroxypropylcellulose, ethylcellulose, triethyl citrate, and talc was prepared using laboratory mixer and preclosed capsules were coated with the so prepared dispersion in a perforated coating pan at about 30°C to obtain the suitable amount of the coating.
The suspension was sprayed over the HPMC capsule bodies in a perforated coating pan at a temperature at about
The coated capsules were opened, filled with 560 mg of the granulate and the tablet, and closed

## Claims

1. A therapeutic system for once or twice-daily administration of amoxicillin and clavulanic acid comprising at least one immediate release amoxicillin/clavulanic acid pharmaceutical composition and at least one floating capsule, wherein the capsule comprises:
- a capsule body which is coated with a coating comprising combination of ethylcellulose and hydroxypropylcellulose,
- a coated or uncoated capsule cap,
- a granulate comprising hydroxypropylmethyl cellulose and amoxicillin, and
- at least one tablet comprising amoxicillin.

2. The therapeutic system of claim 1, wherein amoxicillin is in the form of trihydrate.

3. The therapeutic system of claim 1, wherein clavulanic acid is in the form of potassium clavulanate.

4. The therapeutic system of claim 1 comprising from 800 to 4000 mg of amoxicillin and from 50 to 250 mg of clavulanic acid.

5. The therapeutic system of claims 1 and 4 comprising 1600 mg of amoxicillin and 125 mg of clavulanic acid.

6. The therapeutic system of claim 1, wherein the ratio of amoxicillin and clavulanic acid is 3:1 to 35:1.

7. The therapeutic system of claims 1 and 6 wherein the ratio amoxicillin and clavulanic acid is about 12:1 to about 13:1.

8. The therapeutic system of claim 1, wherein at least one immediate release amoxicillin/clavulanic acid pharmaceutical compositions is selected from the group consisting of a immediate release tablet, film coated tablet, dispersible tablet, effervescent tablet, chewing tablet, capsule, pellets, granulate, suspension.

9. The therapeutic system of claims 1 and 8 wherein the immediate release pharmaceutical composition is a film coated tablet.

10. The therapeutic system of claims 1 and 8 wherein the immediate release pharmaceutical composition is a dispersible tablet.

11. The therapeutic system of claim 1, wherein at least one immediate release pharmaceutical composition comprises from 300 to 2000 mg of amoxicillin and from 50 to 250 mg of clavulanic acid.

12. The therapeutic system of claims 1 and 11, wherein the immediate release pharmaceutical composition comprises 1050 mg of amoxicillin and 125 mg of clavulanic acid.

13. The therapeutic system of claim 1, wherein the immediate release pharmaceutical composition comprises absorption enhancers of amoxicillin from the gastrointestinal tract.

14. The therapeutic system of claim 1, wherein at least one floating capsule comprises from 500 mg to 4000 mg of amoxicillin.

15. The therapeutic system of claims 1 and 14, wherein the floating capsule comprises from 550 mg of amoxicillin.

16. The therapeutic system of claim 1, wherein the floating capsule comprises absorption enhancers of amoxicillin from the gastrointestinal tract.

17. The therapeutic system of claim 1 providing the plasma concentrations of amoxicillin of 4 µg/mL for longer than 7 hours within the dosing interval.

18. The therapeutic system of claim 1 providing the plasma concentrations of amoxicillin of 8 µg/mL for longer than 3.5 hours within the dosing interval.

19. The therapeutic system of claim 1 providing the plasma concentrations of amoxicillin of 1 µg/mL for longer than 12 hours within the dosing interval.

20. The therapeutic system of claim 1 providing the plasma concentrations of amoxicillin of 1 µg/mL for longer than 7.5 hours in the 12-hour dosing interval.

21. The therapeutic system of claim 1 comprising a film coated tablet comprising 1050 mg of amoxycillin and 125 mg of clavulanic acid and a floating capsule comprising 550 mg of amoxicillin.

22. A floating capsule according to claim 1 comprising a capsule body which is coated with a coating comprising combination of ethylcellulose and hydroxypropylcellulose, a coated or uncoated capsule cap, at least one tablet comprising amoxicillin, and a granulate comprising amoxicillin and hydroxypropylmethyl cellulose.

23. The floating capsule of claim 22 which is based on hydroxypropylmethylcellulose.

24. The floating capsule of claim 22, in which the granulate comprises the absorption enhancers of amoxicillin from the gastrointestinal tract.

25. The floating capsule of claim 22, wherein the tablet comprises the absorption enhancers of amoxicillin from the gastrointestinal tract.

26. The floating capsule of claim 22 wherein the granulate and the tablet comprise the absorption enhancers of amoxicillin from the gastrointestinal tract.

27. The use of therapeutic system of claim 1 in the manufacturing of a medicament for the treatment of bacterial infections, the medicament is being administered once or twice daily.

## Patentansprüche

1. Therapeutisches System für eine einmal oder zweimal tägliche Verabreichung von Amoxicillin und Clavulansäure, umfassend wenigstens eine unmittelbar freisetzende pharmazeutische Zusammensetzung von Amoxicillin/Clavulansäure und wenigstens eine Schwimmkapsel, wobei diese Kapsel umfasst:
- einen Kapselkörper, der mit einer Beschichtung versehen ist, die eine Kombination von Ethylcellulose und Hydroxypropylcellulose umfasst,
- eine beschichtete oder unbeschichtete Kapselkappe,
- ein Granulat, umfassend Hydroxypropylmethylcellulose und Amoxicillin, und
- wenigstens eine Amoxicillin enthaltende Tablette.

2. Therapeutisches System nach Anspruch 1, worin Amoxicillin in der Form eines Trihydrats vorliegt.

3. Therapeutisches System nach Anspruch 1, worin Clavulansäure in der Form von Kaliumclavulanat vorliegt.

4. Therapeutisches System nach Anspruch 1, umfassend 800 bis 4000 mg Amoxicillin und 50 bis 250 mg Clavulansäure.

5. Therapeutisches System nach Anspruch 1 bis 4, umfassend 1600 mg Amoxicillin und 125 mg Clavulansäure.

6. Therapeutisches System nach Anspruch 1, worin das Verhältnis von Amoxicillin und Clavulansäure 3:1 bis 35:1 beträgt.

7. Therapeutisches System nach Anspruch 1 und 6, worin das Verhältnis von Amoxicillin und Clavulansäure etwa 12:1 bis etwa 13:1 beträgt.

8. Therapeutisches System nach Anspruch 1, worin wenigstens eine unmittelbar freisetzende pharmazeutische Zusammensetzung von Amoxicillin/Clavulansäure ausgewählt ist aus der Gruppe, die besteht aus einer unmittelbar freisetzenden Tablette, filmbeschichteten Tablette, dispersiblen Tablette, Brausetablette, Kautablette, Kapsel, Pellets, einem Granulat und einer Suspension.

9. Therapeutisches System nach Anspruch 1 und 8, worin die unmittelbar freisetzende pharmazeutische Zusammensetzung eine filmbeschichtete Tablette ist.

10. Therapeutisches System nach Anspruch 1 und 8, worin die unmittelbar freisetzende pharmazeutische Zusammensetzung eine dispersible Tablette ist.

11. Therapeutisches System nach Anspruch 1, worin die wenigstens eine unmittelbar freisetzende pharmazeutische Zusammensetzung 300 bis 2000 mg Amoxicillin und 50 bis 250 mg Clavulansäure umfasst.

12. Therapeutisches System nach Anspruch 1 und 11, worin die unmittelbar freisetzende pharmazeutische Zusammensetzung 1050 mg Amoxicillin und 125 mg Clavulansäure umfasst.

13. Therapeutisches System nach Anspruch 1, worin die unmittelbar freisetzende pharmazeutische Zusammensetzung Absorptionsverstärker von Amoxicillin aus dem Gastrointestinaltrakt umfasst.

14. Therapeutisches System nach Anspruch 1, worin die wenigstens eine Schwimmkapsel 500 mg bis 4000 mg Amoxicillin umfasst.

15. Therapeutisches System nach Anspruch 1 und 14, worin die Schwimmkapsel 550 mg Amoxicillin umfasst.

16. Therapeutisches System nach Anspruch 1, worin die Schwimmkapsel Absorptionsverstärker von Amoxicillin aus dem Gastrointestinaltrakt umfasst.

17. Therapeutisches System nach Anspruch 1, das für Plasmakonzentrationen von Amoxicillin von 4 µg/ml für länger als 7 h im Dosierungsintervall sorgt.

18. Therapeutisches System nach Anspruch 1, das für Plasmakonzentrationen von Amoxicillin von 8 µg/ml für länger als 3,5 h im Dosierungsintervall sorgt.

19. Therapeutisches System nach Anspruch 1, das für Plasmakonzentrationen von Amoxicillin von 1 µg/ml für länger als 12 h im Dosierungsintervall sorgt.

20. Therapeutisches System nach Anspruch 1, das für Plasmakonzentrationen von Amoxicillin von 1 µg/ml für länger als 7,5 h im 12-stündigen Dosierungsintervall sorgt.

21. Therapeutisches System nach Anspruch 1, umfassend eine filmbeschichtete Tablette, die 1050 mg Amoxicillin und 125 mg Clavulansäure und eine Schwimmkapsel umfasst, die 550 mg Amoxicillin enthält.

22. Schwimmkapsel nach Anspruch 1, umfassend einen Kapselkörper, der mit einer Beschichtung versehen ist, die eine Kombination von Ethylcellulose und Hydroxypropylcellulose, eine beschichtete oder unbeschichtete Kapselkappe, wenigstens eine Amoxicillin enthaltende Tablette und ein Granulat von Amoxicillin und Hydroxypropylmethylcellulose umfasst.

23. Schwimmkapsel nach Anspruch 22, die auf Hydroxypropylmethylcelllulose basiert.

24. Schwimmkapsel nach Anspruch 22, deren Granulat Absorptionsverstärker von Amoxicillin aus dem Gastrointestinaltrakt umfasst.

25. Schwimmkapsel nach Anspruch 22, worin die Tablette die Absorptionsverstärker von Amoxicillin aus dem Gastrointestinaltrakt umfasst.

26. Schwimmkapsel nach Anspruch 22, worin das Granulat und die Tablette die Absorptionsverstärker von Amoxicillin aus dem Gastrointestinaltrakt umfassen.

27. Verwendung eines therapeutischen Systems nach Anspruch 1 zur Herstellung eines Arzneimittels für die Behandlung von bakteriellen Infektionen, wobei dieses Arzneimittel einmal oder zweimal täglich verabreicht wird.

## Revendications

1. Système thérapeutique pour l'administration une ou deux fois par jour d'amoxicilline et d'acide clavulanique, comprenant au moins une composition pharmaceutique à base d'amoxicilline/acide clavulanique à libération immédiate et au moins une capsule flottante, dans lequel la capsule comprend :
- un corps de capsule qui est revêtu d'un enrobage comprenant une combinaison d'éthylcellulose et d'hydroxypropylcellulose,
- une coiffe de capsule revêtue ou non revêtue,
- un granulé comprenant de l'hydroxypropylméthylcellulose et de l'amoxicilline, et
- au moins un comprimé comprenant de l'amoxicilline.

2. Système thérapeutique selon la revendication 1, dans lequel l'amoxicilline est sous forme de trihydrate.

3. Système thérapeutique selon la revendication 1, dans lequel l'acide clavulanique est sous forme de clavulanate de potassium.

4. Système thérapeutique selon la revendication 1, comprenant de 800 à 4000 mg d'amoxicilline et de 50 à 250 mg d'acide clavulanique.

5. Système thérapeutique selon les revendications 1 et 4, comprenant 1600 mg d'amoxicilline et 125 mg d'acide clavulanique.

6. Système thérapeutique selon la revendication 1, dans lequel l'amoxicilline et l'acide clavulanique sont dans un rapport de 3/1 à 35/1.

7. Système thérapeutique selon les revendications 1 et 6, dans lequel l'amoxicilline et l'acide clavulanique sont dans un rapport d'environ 12/1 à environ 13/1.

8. Système thérapeutique selon la revendication 1, dans lequel au moins une composition pharmaceutique à base d'amoxicilline/acide clavulanique à libération immédiate est choisie dans le groupe constitué par un comprimé, un comprimé pelliculé, un comprimé dispersible, un comprimé effervescent, un comprimé à croquer, une capsule, des pastilles, un granulé, une suspension, à libération immédiate.

9. Système thérapeutique selon les revendications 1 et 8, dans lequel la composition pharmaceutique à libération immédiate est un comprimé pelliculé.

10. Système thérapeutique selon les revendications 1 et 8, dans lequel la composition pharmaceutique à libération immédiate est un comprimé dispersible.

11. Système thérapeutique selon la revendication 1, dans lequel au moins une composition pharmaceutique à libération immédiate comprend de 300 à 2000 mg d'amoxicilline et de 50 à 250 mg d'acide clavulanique.

12. Système thérapeutique selon les revendications 1 et 11, dans lequel la composition pharmaceutique à libération immédiate comprend 1050 mg d'amoxicilline et 125 mg d'acide clavulanique.

13. Système thérapeutique selon la revendication 1, dans lequel la composition pharmaceutique à libération immédiate comprend des agents stimulant l'absorption d'amoxicilline au niveau du tractus gastro-intestinal.

14. Système thérapeutique selon la revendication 1, dans lequel au moins une capsule flottante comprend de 500 mg à 4000 mg d'amoxicilline.

15. Système thérapeutique selon les revendications 1 et 14, dans lequel la capsule flottante comprend à partir de 550 mg d'amoxicilline.

16. Système thérapeutique selon la revendication 1, dans lequel la capsule flottante comprend des agents stimulant l'absorption d'amoxicilline au niveau du tractus gastro-intestinal.

17. Système thérapeutique selon la revendication 1, fournissant les concentrations plasmatiques d'amoxicilline de 4 µg/ml pendant plus de 7 heures dans l'intervalle entre deux doses successives.

18. Système thérapeutique selon la revendication 1, fournissant les concentrations plasmatiques d'amoxicilline de 8 µg/ml pendant plus de 3 heures et demie dans l'intervalle entre deux doses successives.

19. Système thérapeutique selon la revendication 1, fournissant les concentrations plasmatiques d'amoxicilline de 1 µg/ml pendant plus de 12 heures dans l'intervalle entre deux doses successives.

20. Système thérapeutique selon la revendication 1, fournissant les concentrations plasmatiques d'amoxicilline de 1 µg/ml pendant plus de 7 heures et demie dans l'intervalle de 12 heures entre deux doses successives.

21. Système thérapeutique selon la revendication 1, comprenant un comprimé pelliculé comprenant 1050 mg d'amoxicilline et 125 mg d'acide clavulanique et une capsule flottante comprenant 550 mg d'amoxicilline.

22. Capsule flottante selon la revendication 1, comprenant un corps de capsule qui est revêtu d'un enrobage comprenant une combinaison d'éthylcellulose et d'hydroxypropylcellulose, une coiffe de capsule revêtue ou non revêtue, au moins un comprimé comprenant de l'amoxicilline, et un granulé comprenant de l'amoxicilline et de l'hydroxypropylméthylcellulose.

23. Capsule flottante selon la revendication 22, qui est à base d'hydroxypropylméthylcellulose.

24. Capsule flottante selon la revendication 22, dans laquelle le granulé comprend les agents stimulant l'absorption d'amoxicilline au niveau du tractus gastro-intestinal.

25. Capsule flottante selon la revendication 22, dans laquelle le comprimé comprend les agents stimulant l'absorption d'amoxicilline au niveau du tractus gastro-intestinal.

26. Capsule flottante selon la revendication 22, dans laquelle le granulé et le comprimé comprennent les agents stimulant l'absorption d'amoxicilline au niveau du tractus gastro-intestinal.

27. Utilisation du système thérapeutique selon la revendication 1 dans la fabrication d'un médicament destiné à traiter des infections bactériennes, le médicament étant administré une ou deux fois par jour.
